# EUROPEAN PATENT APPLICATION

(11) **EP 4 684 746 A1**
(43) Date of publication of application: **28.01.2026**
(21) Application number: 24191052.0
(22) Date of filing: 26.07.2024
(51) Int. Cl.: A61B 17/70

(54) **COUPLING UNIT AND A SCREW SYSTEM COMPRISING THE COUPLING UNIT**

(71) Applicant: AM Solutions Holding B.V., 2595 AM The Hague (NL)
(72) Inventor: SAJADI, Banafsheh, 2495 AM The Hague (NL); AARTS, Sanne, 2595 AM The Hague (NL); CAINE, Jonathan Andrew, 2595 AM The Hague (NL); AHMADI, Seyed Mohammad, 2595 AM The Hague (NL)
(74) Representative: Hepp Wenger Ryffel AG

(57) **Abstract**

The invention relates to a coupling unit (101) for coupling a longitudinal element (5) to a bone anchoring element (6), in particular for a bone screw system. The coupling unit comprises a tulip element (2) including a receiving area (33) for receiving a head (61) of the bone anchoring element (6), an upper section (34), and at least one aperture (31, 32) in the upper section (34) for receiving the longitudinal element (5). The coupling unit (101) includes a locking mechanism (4) comprising a radially expandable retention ring (42) and a fixation cap (41) which is placeable in the receiving area (33). The retention ring (42) has a retention surface for axially retaining the head (61) and a passage (422) for passing through the head (61). The locking mechanism (4) is convertible between a pass-through state (43) and a locking state (44) by axially moving the fixation cap (41) along the through hole (3). In the pass-through state (43), the retention ring (42) assumes a radially expanded or expandable configuration in an at least partially circumferential space (8) of the receiving area (33) and is configured for passing the head (61) of the bone anchoring element (6) through the passage (422) of the retention ring (42). In the locking state (44), the retention ring (42) of the locking mechanism (4) assumes a radially restricted position such that the retention surface engages at least a lower part of the head (61) of the bone anchoring element (6) such that passing of the head (61) of the bone anchoring element (6) through the passage (422) is prevented. The fixation cap (41) has a first engagement surface (411) and the retention ring has a second engagement surface (421) configured such that upon axial movement of the fixation cap (41) towards the retention ring (42), the first and second engagement surfaces (411, 421) engage with each other thereby converting the locking mechanism (4) from the pass-through state to the locking state.

## Description

The invention relates to a coupling unit for coupling a longitudinal element to a bone anchoring element and a screw system comprising the coupling unit and a bone anchoring element.

Reliable bone fixation by a coupling unit is detrimental for an array of surgical procedures, in particular for fixing bone screws in a specific position and orientation, e.g. during spinal surgery. This fixation via a coupling unit has to ensure sufficient adaptability to a complex anatomy and dynamic loads, proper alignment to other components, sufficient stability, and load distribution for treatment of fractures, in particular for treatment of vertebrae fractures. The bone anchoring elements and the coupling unit have to be adaptable to the pathologic conditions and anatomies, e.g. by enabling a multi-directional orientation of polyaxial screws.

US 8,556,938 B2 discloses a polyaxial bone anchor with a nonpivotable retainer and a pop-on shank to provide a friction fit. US 8,556,938 B2 further discloses an assembly having an expansion chamber having two cylindrical surfaces with different cross-sectional dimensions and a retainer which is movable between the two cylindrical surfaces to provide a locking mechanism for a shank body. The retainer enables a friction fit of the shank body by a rod being axially pushed on a compression insert via a closure structure. Thereby, the compression insert moves the screw downwardly such that the screw engages the retainer and pushes it to the lower cylindrical surface having a smaller cross-sectional dimension such that the retainer engages the shank body in the friction fit.

However, the prior art lacks a solution for a coupling unit which is reliable, simple, and compact. Moving the retainer, in particular between different cylindrical grooves, requires a complex and axially elongated design. This design may also increase wear and tear and carries a risk of unintentional wedging such that a retainer/retention ring cannot return to the respective cylindrical surface. In particular, a simple removal of the screw head may not be possible since retracting the screw may also retract the retainer to the lower cylindrical surface of the expansion chamber.

It is an object of the present invention to overcome these and other disadvantages of the prior art and provide a coupling unit and a screw system which solve the above-mentioned technical problems by providing a simple, reliable, and cost-effective solution. In particular, the present invention shall enable a seamless integration of the coupling unit in a screw system, a streamlined and enhanced operation of the coupling unit, and compact design for receiving and locking a bone anchoring element in the coupling unit. Moreover, the invention shall provide a coupling unit which facilitates and simplifies retraction/removal of a head from the coupling unit.

This object is solved by a coupling unit and screw system according to the independent claims. Further embodiments result from the dependent claims.

The coupling unit is configured for coupling a longitudinal element to a bone anchoring element, in particular for a screw system, preferably a screw system for an intra-vertebral implant. The coupling unit comprises a tulip element which has an axially extending through hole. The through hole includes a receiving area for receiving a head of the bone anchoring element, in particular a bone screw, an upper section, preferably having an inner thread, and at least one aperture in the upper section for receiving the longitudinal element. The coupling unit comprises a locking mechanism including a radially expandable retention ring and a fixation cap which is placeable in the receiving area. The retention ring has a retention surface for axially retaining the head of the bone anchoring element and a passage for passing the head of the bone anchoring element through the retention ring. The locking mechanism is convertible between a pass-through state and a locking state by axially moving the fixation cap along the through hole. In the pass-through state, the retention ring assumes a radially expanded or expandable configuration in an at least partially circumferential space of the receiving area and is configured for passing the head of the bone anchoring element through the passage of the retention ring. In the locking state, the retention ring of the locking mechanism assumes a radially restricted position such that the retention surface engages at least a lower part of the head of the bone anchoring element such that passing of the head of the bone anchoring element through the passage is prevented. The fixation cap has a first engagement surface and the retention ring has a second engagement surface configured such that upon axial movement of the fixation cap towards the retention ring, the first and second engagement surfaces engage with each other. Thereby, the engagement surfaces convert the locking mechanism from the pass-through state to the locking state.

The engagement of the first and second engagement surface may be configured, in particular by being sized and shaped accordingly, to convert the locking mechanism without the involvement of any other additional parts such as the tulip element.

The fixation cap and the retention ring, and in particular the tulip element, may be arranged concentrically with respect to each other.

The tulip element may have a height of 10 mm to 25 mm, in particular 12 mm to 20 mm, preferably 14 mm to 18 mm. The tulip element may have a maximum width of 5 mm to 14 mm, in particular 7 mm to 11 mm, preferably 8 mm - 10 mm.

These dimensions enable a compact design of the coupling unit due to the space-saving construction of the locking mechanism.

The expandable configuration of the retention ring may be configured such that the passage for receiving the head can be widened by passing the head through it. This may allow that the head may be received/retracted through the retention ring in the pass-through state while ensuring that the head is retained by a spring force of the retention ring once received. Thereby, inadvertent removal of the head, e.g. due to minor movements of the coupling unit may be avoided.

The retention ring may be arranged in the at least partially circumferential space in an axially immovable position.

This enables a more compact design and reduces the complexity of the coupling unit by minimizing the number of moving parts and thereby increases reliability. The at least partially circumferential space may comprise or consist of a cylindrical recess.

The retention ring may have an at least partially circumferential radial protrusion which is lowered in the at least partially circumferential space in the pass-through and locking state such that the retention ring is immovable in the axial direction. The at least partially circumferential radial protrusion and the at least partially circumferential space may be correspondingly formed, in particular in a form-fitting manner.

Alternatively, the retention ring may be axially movable with respect to the tulip element. In this case, the receiving area/the at least partially circumferential space may have an at least partially conical shape to constrict a radial dimension of the retention ring at a specific axial position.

The engagement surface of the fixation cap may be arranged at least partially circumferentially within the retention ring in the locking state.

The engagement surface of the fixation cap may be configured to engage both the retention ring and the head of the bone anchoring element in the locking state. The engagement surface may have a wedge shape. The engagement surface of the fixation cap may be configured to radially expand an upper section of the retention ring, in particular having a sleeve-like shape, when engaging both the retention ring and the bone anchoring element.

The retention ring of the locking mechanism in the locking state may have a radially expanded upper section and a radially restricted lower section. The lower section may be restricted by the shape of the tulip element, in particular an at least partially conical shape of the tulip element, in the locking state.

The first and the second engagement surface may include at least one bevel surface inclined with respect to the axis of the coupling unit. The first and the second engagement surface may each include a first and a second bevel surface, respectively.

The bevel surface and the engagement surface without bevel surface or the first and second bevel surfaces may be formed correspondingly to each other. A first bevel surface of the first engagement surface of the fixation cap may face radially inwardly. A second bevel surface of the second engagement surface of the retention ring may face radially outwardly.

The at least one bevel surface comprise or consist of a straight and/or a round bevel.

The outwardly/inwardly facing bevel surface may have an angle with respect to the axial direction between 1° to 89°, in particular between 20° to 70°, preferably between 35° to 55°, away or towards the longitudinal axis.

The at least one bevel surface may enable a seamless conversion between the pass-through state and the locking state. The bevel surface and the engagement surface without bevel surface or the first and second bevel surface engaging each other may be configured for exerting a radially inwardly directed force on the retention ring. Alternatively, the at least one bevel surface may be configured for exerting a radially outwardly directed force on the retention ring.

The retention ring may have a radially outer annular part received in the at least partially circumferential space and a radially inner annular part including the bevel surface.

The tulip element may comprise an abutment surface engageable with the fixation cap in the receiving area to prevent movement of the fixation cap in an upward axial direction beyond a predetermined axial position.

This enables reliable placement of the locking mechanism in the receiving area without a risk of involuntarily removing the fixation cap and/or the retention ring. Thereby, the locking mechanism stays reliably placed within the through hole in the pass-through and in the locking state.

The fixation cap may be shaped and sized to enclose the retention ring at least partially along a circumferential direction of the retention ring to retain the retention ring in the restricted position in the locking state.

This enables the fixation cap, in particular the engagement surface of the fixation cap, to retain the retention ring by engaging the engagement surface, in particular the bevel surface, of the retention ring.

The retention ring may be elastically deformable and/or may be radially compressed in the locking state.

This enables to provide retention ring in the radially restricted position which is configured to engage the head to fix the position/orientation of the bone anchoring element.

The retention ring may have an upwardly protruding ridge, in particular a cylindrical upwardly protruding ridge. The upwardly protruding ridge may be at least partially enclosed or enclosable along the circumferential direction by the fixation cap in the locking state. The upwardly protruding ridge may comprise the second bevel surface, in particular the radially outwardly facing second bevel surface, of the second engagement surface.

The second bevel surface may be shaped and sized to radially engage and move the fixation cap in an upward axial direction when the head is passed through the passage of the retention ring when removing and/or receiving the head.

This allows to provide a compact and automatic locking mechanism. This also enables to ensure that the head can be reliably passed through the retention ring and received in the coupling unit without necessitating any additional spring elements, i.e. solely by interaction between the retention ring and the fixation cap with the head.

Alternatively or additionally, the coupling unit may comprise a spring element such that the locking mechanism automatically assumes the pass-through state when no external forces are exerted on the locking mechanism and/or if the shank head is received within the receiving chamber. This may e.g. be achieved by the spring element pre-tensioning the fixation cap or the retention ring of the locking mechanism towards the expandable or expanded position/state of the retention ring.

The at least partially circumferential space may have an unoccupied section in the pass-through state and the retention ring may be radially expandable by advancing and/or retracting the head of the bone anchoring element through the passage such that the retention ring is radially expanded into the unoccupied section.

This enables the retention ring to be arranged within the circumferential space in the expanded and non-expanded state, enabling a more compact design.

A compression force of the retention ring in the constricted position/shape may be sufficient to retain the head in the receiving chamber even in the pass-through state by engaging the lower section of the head.

The locking mechanism may be configured such that the retention ring cannot be radially expanded into the unoccupied section in the locking state.

The retention ring may be a circlip or a collet and may in particular comprise at least one gap or split.

This enables a simple and reliable design of a retention ring which is radially expandable.

The retention ring may have an undulated shape. The retention ring may comprise a plurality of separate segments or be formed in one piece.

The retention ring preferably comprises or consists of a metal, a ceramic, a plastic, and/or a composite material. In particular the retention ring may comprise or consist of steel, titanium, nitinol, brass, aluminium, polyamide, acetal resin, polyetheretherketone, carbon fiber or glass fiber reinforced polymer, alumina, and/or zirconia.

Alternatively or additionally, the retention ring may comprise or consist of an elastic material, in particular natural rubber, synthetic rubber, preferably ethylene propylene diene monomer rubber, silicone, thermoplastic elastomers, and/or synthetic elastomers.

The fixation cap and the at least one aperture may overlap at least partially in the axial direction in the pass-through state of the locking mechanism.

This enables a longitudinal element which extends through the at least one aperture, in particular two opposing apertures, to contact the fixation cap. Thereby, the fixation cap may be moved by an axial force exerted through the longitudinal element in the aperture to move the fixation cap and convert the locking mechanism between the pass-through and the locking state.

The locking mechanism may be shaped and sized such that if an axially downwardly directed force is exerted on the fixation cap towards the retention ring, the locking mechanism is converted to the locking state and the head cannot be passed through the passage. The locking mechanism may be shaped and sized that if no axially directed force is exerted on the fixation cap, the locking mechanism is converted to the pass-through state and the head is configured to be advanced and/or retracted through the passage.

This enables an automated locking mechanism which automatically enables advancement/retraction of the head without the axially downwardly directed force while locking the head reliably if, e.g. a set screw and the longitudinal element exert the axially downwardly directed force.

The fixation cap may have an axial through hole such that a driver for the head of the bone anchoring element is insertable through the axial through hole.

This may enable reliably actuating the head of the bone anchoring element through the axial through hole of the fixation cap and the passage of the retention ring, even though the locking mechanism is arranged in the receiving area of the coupling unit.

The fixation cap may have a receiving chamber which includes an at least partially spherical inner surface for at least partly frictionally engaging at least an upper part of the head of the bone anchoring element.

The head may have a spherical outer surface which is formed correspondingly to the spherical inner surface of the fixation cap.

The at least partially spherical inner surface may enable polyaxial tiltability of the bone anchoring element while the head remains in the receiving chamber.

The upper section may have at least two apertures for receiving the longitudinal element which are arranged on opposing sides of the tulip element and preferably axially extend to an end of the upper section.

This enables the longitudinal element to be received through both apertures which are arranged on the opposing sides. The apertures extending to the end of the upper section allows the longitudinal element to be inserted into the two apertures in an axial manner.

The locking mechanism in the pass-through state may be configured for engaging the head of the bone anchoring element such that the head of the bone anchoring element is tiltable polyaxially. The locking mechanism, in particular the fixation cap and/or the retention ring may frictionally engage the head of the bone anchoring element in the locking state such that the bone anchoring element is non-tiltable.

This enables to align the coupling unit in the pass-through state with respect to the bone anchoring element and lock a position/orientation of the coupling unit in the locking state with respect to the bone anchoring element.

The locking mechanism in the pass-through state may be configured to allow movement of at least 30°, in particular at least 50°, preferably at least 60°, of the bone anchoring element with respect to the longitudinal axis of the coupling unit.

Another aspect of the invention relates to a screw system, in particular for an intra-vertebral implant, comprising at least one previously described coupling unit and at least one bone anchoring element, in particular at least one bone screw.

This enables to provide a screw system that is optimally matched in terms of function and structure.

The bone anchoring element may comprise or consist of a bone screw or a bone implant. The bone anchoring elements may comprise a radially expandable section similarly to a dowel. The bone anchoring element may be a bone screw for treatment of vertebral compression fractures. The bone screw or bone implant may be shaped and sized to be inserted into a vertebral body or a pedicle. The bone screw or bone implant may be shaped and sized for stabilizing the vertebrae in a procedure such as a spinal fusion, a treatment of a vertebral fracture, or a scoliosis correction.

The screw system may be configured to be used without bone cement or with bone cement.

The head may have a maximum cross-sectional dimension which is larger than the cross-sectional dimension of the passage of the retention ring in a non-actuated state.

The retention ring may be shaped and sized to assume the radially expandable configuration in the pass-through state if no forces are exerted on the retention ring, i.e. the retention ring is in the non-actuated state. The retention ring may be shaped and sized such that, if the head of the bone anchoring element is passed through the passage of the retention ring, the retention ring assumes the retention ring is converted to a radially expanded configuration. In the radially expanded configuration, the retention ring may exert an inwardly directed force, e.g. on the head of the bone anchoring element.

The retention ring may be shaped and sized to assume a constricted position/shape in the locking state in which the passage is constricted to a smaller radial dimension than in the expanded or expandable configuration of the retention ring.

The bone anchoring element being a bone screw may have a length of 20 mm to 70 mm, in particular 30 mm to 60 mm, preferably 40 mm to 50 mm.

A shaft of the bone screw may have a diameter of 3 mm to 8 mm, in particular 4 mm to 7 mm, preferably 5 mm to 6 mm.

The bone screw may have an upper section comprising a thread and a lower section comprising at least one radially expandable section, in particular a plurality of radially expandable sections.

The tulip element, the fixation cap, and/or the bone anchoring element may comprise or consist of titanium, stainless steel, polyetheretherketone, carbon fibre reinforced polymer, nitinol, ceramic material, tantalum or cobalt-chromium alloys.

The screw system may comprise at least one longitudinal element which is shaped and sized to be received laterally in the through hole, in particular through the at least one aperture, preferably through the two apertures on opposing sides of the tulip element.

This screw system may provide a single, compact unit having mutually optimized components, thereby enhancing surgical efficiency and improving operability of the screw system.

A width of the aperture along a circumferential direction of the tulip may correspond to the width of the longitudinal element. An axial length of the aperture may be larger than the width of the longitudinal element such that the longitudinal element is movable in an axial direction of the at least one aperture. The longitudinal element may be insertable into the at least one aperture from an open end at the end of the upper section of the tulip element.

The longitudinal element is preferably shaped and sized to be received through the two apertures on opposing sides of the through hole such that the longitudinal element engages the locking cap. The overlap between the fixation cap and the aperture may further enable that the longitudinal element engages the fixation cap. The longitudinal element may be arranged to exert an axial force on the fixation cap when moved axially downwardly along the through hole, in particular while laterally extending through the at least one aperture. Thereby, the locking mechanism may be converted from the pass-through state to the locking state by moving the longitudinal element downwardly in the at least one aperture.

The screw system may comprise a set screw which comprises an outer thread which insertable into the inner thread of the tulip element for exerting a downward axial force on the longitudinal element towards the fixation cap, such that the locking mechanism is converted from the pass-through state to the locking state.

The downward axial force exerted on the longitudinal element may enable the longitudinal element being clamped between the fixation cap and the set screw. The set screw may axially move the fixation cap via the longitudinal element towards the retention ring and thereby convert the locking mechanism from the pass-through state to the locking state.

The first engagement surface of the fixation cap may be shaped and sized to engage the second engagement surface during the downward axial movement of the fixation cap and radially restricts the retention ring. The first engagement surface, in particular comprising a first bevel surface, may engage the protruding ridge, in particular a second bevel surface, of the retention ring to radially restrict the retention ring.

Further embodiments of the invention and improvement of the described embodiments will become apparent with the following description of the embodiments.

The invention is now described with reference to certain embodiments and figures which show:
Figure 1A: a perspective exploded view of a screw system comprising the coupling unit according to the invention,
Figure 1B: a perspective view of the composite screw system according to Fig. 1A,
Figure 2A: a cross-sectional view of the coupling unit according to Figs. 1A and 1B,
Figures 2B and 2C: a cross-sectional view of the coupling unit receiving a head of a bone anchoring element in a pass-through state of the locking mechanism,
Figure 2D: a cross-sectional view of the screw system comprising the coupling unit of Figs. 2A - 2C with a longitudinal element which was received within the apertures of a tulip element of the coupling unit which is pushed towards a fixation cap of the coupling unit by a set screw,
Figure 2E: a cross-sectional view of the screw system including a detailed force transmission during conversion to the locking state of the locking mechanism according to Fig. 2D,
Figure 2F: a cross-sectional view of the coupling unit once the longitudinal element was removed such that the head of the bone anchoring element can be removed in the pass-through state of the coupling unit,
Figure 3: a perspective exploded view of an alternative embodiment of the coupling unit according to the invention, and
Figures 4A - 4C: a cross-sectional view of the alternative embodiment of the coupling unit of Fig. 3 receiving a head of a bone anchoring element.

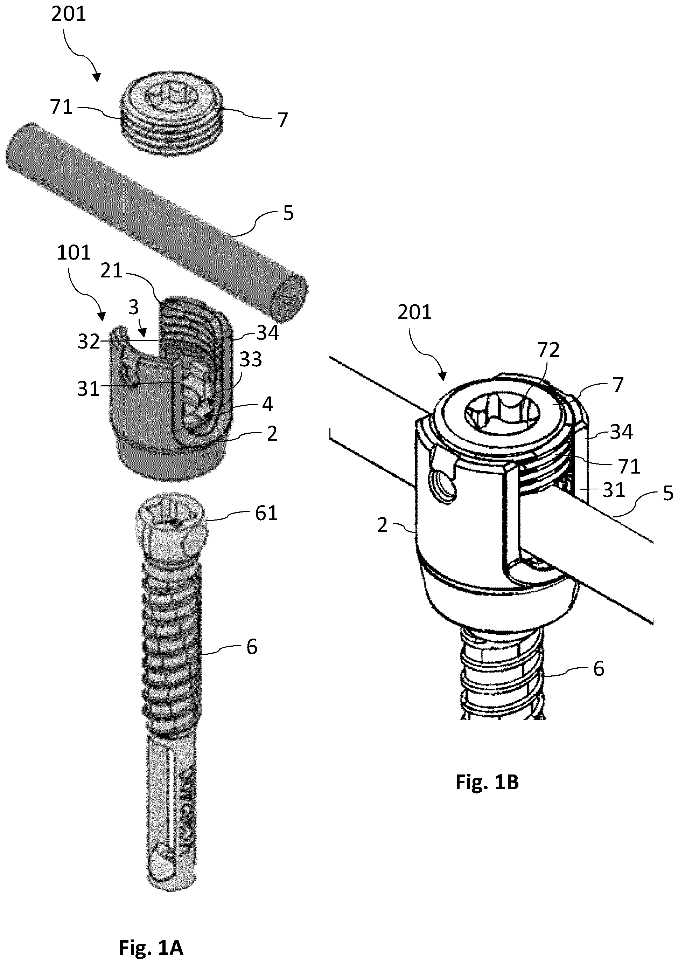

Figure 1A shows a perspective exploded view of a screw system 201 comprising the coupling unit 101. The screw system 201 further includes a set screw 7 which has an outer thread 71, a longitudinal element 5 formed by a bar, and a bone anchoring element 6 formed by a bone screw having a spherical head 61.

The coupling unit 101 includes a tulip element 2 and a locking mechanism 4 which is received within a receiving section 33 of the tulip element 2. An axial through hole 3 extends through the tulip element 2. The tulip element 2 further includes an upper section 34 which has an inner thread 21 which is correspondingly formed to the outer thread 71 of the set screw 7. The tulip element 2 further comprises two apertures 31, 32 which are arranged on opposing lateral sides of the tulip element 2 and extend up to the end of the upper section 34 to form an open shape.

The bar 5 may be axially inserted into the apertures 31, 32 such that it laterally intersects the axial through hole 3 (see Fig. 1B). The locking mechanism 4 is partially arranged adjacent the apertures 31, 32 such that the locking mechanism 4 and the apertures 31, 32 partially overlap in the axial direction of the coupling unit 101.

Figure 1B shows a perspective view of the composite screw system 201 according to Fig. 1A.

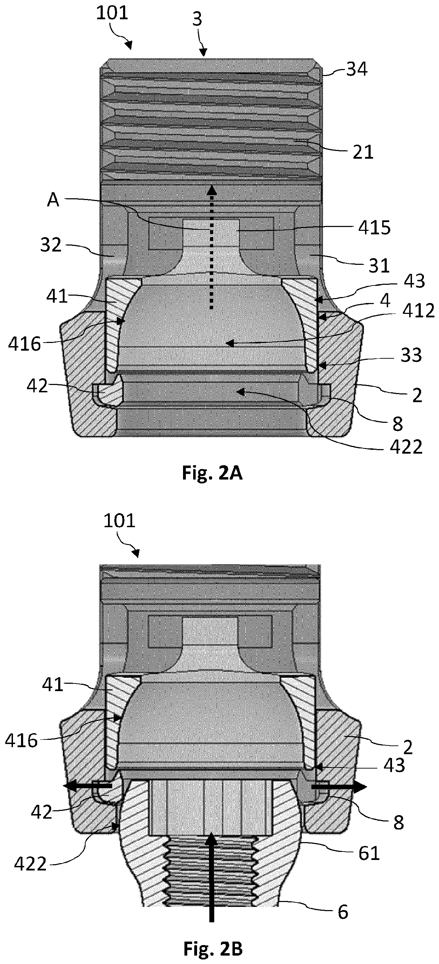

The bar 5 is inserted into the apertures 31, 32 of the coupling unit 101 and contacts the locking mechanism 4. This enables the bar 5 to exert a force on the locking mechanism 4. The set screw 7 in Fig. 1B is inserted into the upper section 34 to clamp and move the bar 5 in a downwardly axial direction towards the locking mechanism 4. This downwardly axial force converts the locking mechanism 4 from a pass-through state to a locking state to lock a position/orientation of the bone anchoring element 6 with respect to the tulip element 2 as will be described in Figs. 2A - 2F in further detail. The set screw 7 comprises an actuation section 72 on its head, e.g. a Torx actuation section, to be actuatable by a driving tool to facilitate rotation of the set screw 7.

Figure 2A shows a cross-sectional view of the coupling unit 101 according to Figs. 1A and 1B. The coupling unit 101 comprises the tulip element 2 with an upper section 34 having an inner thread 21 and two apertures 31, 32 on opposing lateral sides of the tulip element 2. The coupling unit 101 further comprises a locking mechanism 4 including a fixation cap 41 and a retention ring 42.

The locking mechanism 4 of Fig. 2A is in a pass-through state 43. No forces are exerted on the fixation cap 41 such that the fixation cap 41 is at least partially axially movable with respect to the retention ring 42.

The fixation cap 41 has an abutment surface 415 which is configured to engage a recessed section of the tulip element 2 when the fixation cap 41 is arranged within a receiving area 33 of the tulip element 2. This abutment surface 415 limits the movement of the fixation cap 41 in an upward axial direction A beyond a predetermined axial position. The fixation cap 41 is axially downwardly movable towards the retention ring 42 in the receiving area 33.

The fixation cap 41 in Fig. 2A protrudes from the receiving area 33 such that the apertures 31, 32 and the fixation cap 41 overlap in the axial direction of the coupling unit 101.

The fixation cap 41 has an axial through hole 412 and the retention ring 42 has a passage 422 which extend through the fixation cap 41 and retention ring 42 in the axial direction and enable a driver to engage a head of a bone anchoring element through an axial through hole of the tulip element 2, the axial through hole 412 of the fixation cap 41, and the passage 422 of the retention ring 42.

The fixation cap 41 has a partially spherical inner surface which forms a receiving chamber 416 which is formed correspondingly to a head 61 of a bone anchoring element 6 (see Fig. 2B).

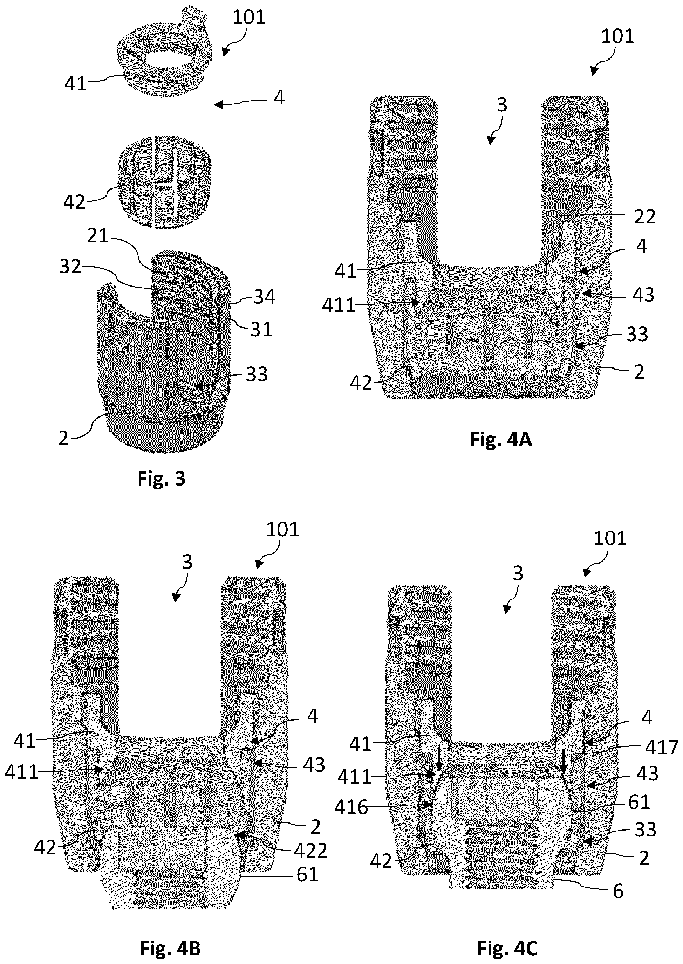

The retention ring 42 in Fig. 2A is arranged partially protruding into a circumferential space 8 which is radially recessed into the tulip element 2 and correspondingly formed to an engagement section of the retention ring 42. The retention ring 42 in this embodiment is formed by a circlip which is radially expandable by having an at least partially open ring shape. The retention ring 42 shown in Figs. 2A - 2F is formed by a one-piece circlip. Alternatively, the retention ring 42 may be formed by a plurality of separated segments and/or in particular have an undulated sleeve shape (see Figs. 3 - 4C).

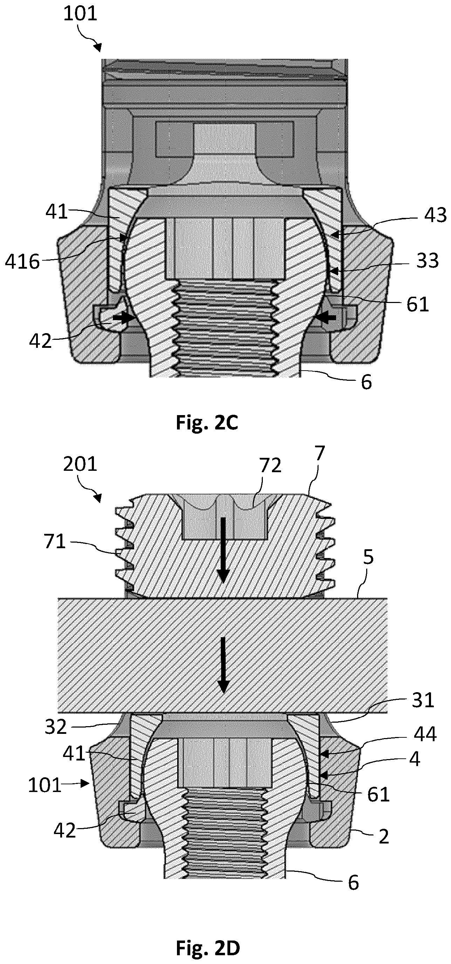

Figures 2B and 2C show a cross-sectional view of the coupling unit 101 of Fig. 2A receiving a head 61 of a bone anchoring element 6 in the pass-through state 43 of the locking mechanism 4.

While passing the head 61 through the passage 422 as shown in Fig. 2B, the retention ring 42 is expanded from a default expandable configuration to a radially expanded configuration which is indicated by the radially outwardly directed arrows. The section of the retention ring 42 which partially protrudes into the circumferential spacing 8 is pushed radially outwardly further into the circumferential spacing 8 while the cross-sectional dimension of the retention ring 42 is expanded. The expanded cross-sectional dimension is large enough to receive the head 61. The retention ring 42 is pretensioned to return to the default radially expandable configuration after the head 61 was received in the receiving chamber 416 of the fixation cap 41 as shown in Fig. 2C. This is shown in Fig. 2C by the inwardly directed arrows. The retention ring 42 in the default expandable configuration engages the lower part of the head 61 of the bone anchoring element 6 with a retention surface to retain the bone anchoring element 6 in a polyaxially movable manner. An outer surface of the head 61 in Fig. 2C, in which the locking mechanism 4 still assumes the pass-through state 43, is held by the retention ring 42 in the receiving area 33 in a removable manner.

Figure 2D shows a cross-sectional view of the screw system 201 comprising the coupling unit 101 with a longitudinal element 5 which was received within the apertures 31, 32 of the tulip element 2. The longitudinal element 5 is pushed towards the fixation cap 41 of the coupling unit 101 by a set screw 7 of the screw system 201 as indicated by the black arrows. The set screw 7 has an actuation section 72 on an upper surface such that an outer thread 71 of the set screw 7 can be advanced into the inner thread 21 (see Fig. 2A) of the tulip element 2 to clamp the longitudinal element 5 via a driving tool. The longitudinal element 5 contacts a top surface of the fixation cap 41 such that the fixation cap 41 is axially movable by pushing the section of the fixation cap 41 which overlaps axially with the apertures 31, 32 downwardly towards the retention ring 42. Thereby, the locking mechanism 4 is converted to a locking state 44 of the locking mechanism 4 such that a head 61 of the bone anchoring element 6 is non-movable with respect to the coupling unit 101.

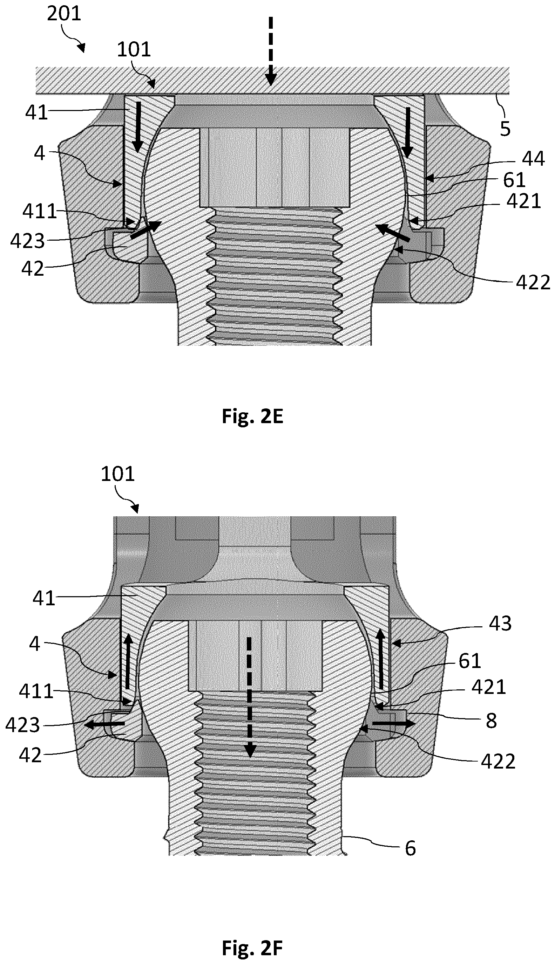

Figure 2E shows a cross-sectional view of the screw system 201 including a detailed force transmission during the conversion from the pass-through state to the locking state 44 of the locking mechanism 4 according to Fig. 2D.

The fixation cap 41 is moved axially downwardly indicated by the downwardly directed black arrows due to a pushing force on the longitudinal element 5 depicted as the dashed black arrow in Fig. 2E. The fixation cap 41 has an inwardly facing bevel surface of a first engagement surface 411.

The retention ring 42 has a cylindrically upwardly protruding ridge 423 which has an outwardly facing bevel surface of a second engagement surface 421.

The first engagement surface 411 of the fixation cap 41 circumferentially encloses the protruding ridge 423 of the retention ring 42 in the locking state 44. Thereby, the retention ring 42 assumes a non-expandable configuration / a radially restricted position. In the locking state 44 the retention ring 42 engages the lower part of the head 61 as indicated by radially inwardly directed arrows originating from the retention ring 42 such that passing the bone anchoring element 6 through the passage 422 is prevented. By this design the downwardly directed force exerted of the longitudinal element 5 on the locking mechanism 4 due to the set screw can thereby automatically convert the locking mechanism 4 from the pass-through state to the locking state 44. The bevel surfaces enable a more seamless conversion of the locking mechanism 4 and an application of an inwardly directed force transmission. Moreover, the insertion and retraction of the head 61 through the retention ring 4 can automatically axially move the fixation cap 41, simplifying insertion and retraction of the head 61. The retention ring 42 and/or the fixation cap 41 engage the head 61 frictionally in such a manner that the axial position of the bone anchoring element 6 is fixed in the locking state 44, i.e. the bone anchoring element 6 is not polyaxially tiltable.

Figure 2F shows a cross-sectional view of the coupling unit 101 once the longitudinal element 5 (see Figs. 2D and 2E) was removed such that the locking mechanism 4 assumes the pass-through state 43 and the head 61 of the bone anchoring element 6 can be removed. By removing the downwardly directed force applied via the longitudinal element 5, the fixation cap 41 is upwardly axially movable again with respect to the retention ring 42.

When the head 61 is moved through the passage 422, the retention ring 42 is radially expanded, i.e. the radially protruding section of the retention ring 42 is pressed into the circumferential spacing 8. This expansion of the retention ring 42 is automatically caused by passing the head 61 through the passage 422 of the retention ring 42.

The outwardly facing bevel surface of the circumferentially protruding ridge 423 of the retention ring 42 engages the engagement surface 411 of the fixation cap 41 which encloses the retention ring 42 when the retention ring 42 expands. The outwardly facing bevel surface of the ridge 423 is coupled to the bevel surface of the engagement surface 411 such that a radial expansion of the retention ring 42 pushes the fixation cap 41 axially upwardly in the pass-through state 43 by removing the head 61. Thereby, the bone anchoring element 6 can be reliably removed, in particularly manually without any auxiliary means, in the pass-through state 43 once the fixation cap 41 is axially movable again. The radial expansion of the retention ring 42 is depicted by the radially outwardly directed arrows, the axial movement of the fixation cap 41 is depicted as the axially upwardly directed arrows, and the removal of the head 61 is depicted as the axially downwardly directed dashed arrow in Fig. 2F.

Figure 3 shows a perspective exploded view of an alternative embodiment of the coupling unit 101. The coupling unit 101 has a similar design and functionality as previously described and comprises a locking mechanism 4 including a fixation cap 41 and a retention ring 42. The locking mechanism 4 is also placeable within a receiving area 33 of a tulip element 2 which has an upper section 34 with an inner thread 21 and two apertures 31, 32 on opposing lateral sides.

Contrary as previously described, the retention ring 42 of Fig. 3 has a sleeve shape. The retention ring 42 has is a one-piece retention ring 42 which has a circumferentially closed shape. The retention ring 42 has an undulating shape, i.e. a series of alternating peaks and troughs, such that the retention ring 42 is radially expandable and radially compressible.

Figures 4A - 4C show a cross-sectional view of the alternative embodiment of the coupling unit 101 of Fig. 3 receiving a head 61 of a bone anchoring element 6.

Figure 4A shows that the fixation cap 41 and the retention ring 42 are placed within the receiving area 33 within a through hole 3 of the tulip element 2. The fixation cap 41 is movable in an axial direction of the coupling unit 101 and the range of movement is limited by an abutment surface 22 of the tulip element 2. The tulip element 2 forms an axially extending recess for guiding a radial projection of the fixation cap 41 which is limited by the abutment surface 22 which limits axial movement of the radial projection beyond a predetermined axial position.

The fixation cap 41 of Figs. 4A - 4C includes downwardly protruding engagement surface 411. The engagement surface 411 is at least partially arranged circumferentially enclosed by the retention ring 42.

Figure 4B shows that the head 61 of the bone anchoring element 6 can be advanced through a passage 422 of the retention ring 42 when the locking mechanism 4 is in a pass-through state 43. When advancing the head 61 axially upwardly through the passage 422, the retention ring 42 is moved axially upwardly toward the fixation cap 41. The retention ring 42 is radially expanded from an expandable configuration having a smaller radial dimension to an expanded configuration having a larger radial dimension when the head 61 is advanced through the passage 422. The head 61 has a larger radial dimension than the retention ring 42 in the expandable configuration.

Figure 4C shows that the head 61 was received within a receiving chamber 416 which forms a spherical surface and encloses an outer spherical surface of the head 61. The engagement surface 411 of the fixation cap 41 is configured to engage the head 61 on an inwardly facing side and the retention ring 42 on an outwardly facing side. The engagement surface 411 has a wedge shape which is at least partially arranged/arrangeable between the retention ring 42 and the head 61. In the pass-through state 43 in Fig. 4C, the bone anchoring element 6 is polyaxially movable while the head 61 remains securely within the receiving chamber 416. The retention ring 42 is pretensioned such that a lower section of the head 61 can be securely held in the receiving chamber 416.

The fixation cap 41 is actuatable similarly as previously described, i.e. by placing a longitudinal element 5, e.g. a bar, extending laterally through the apertures 31, 32 (see Fig. 3) and clamping the longitudinal element 5 by tightening a set screw 7 such that the longitudinal element 5 axially moves the fixation cap 41 downwardly (see Figs. 2D and 2E). This is indicated by the axially downwardly facing arrows in Fig. 4C. By moving the fixation cap 41 axially downwardly, the coupling unit 101 can be converted from the pass-through state 43 to a locking state in which the bone anchoring element 6 is non-removable.

Based on the downward movement, a circumferential step 417 on a radially outwardly facing side of the fixation cap 41 pushes the retention ring 42 also axially downwardly. The receiving area 33 of the through hole 3 has a conically formed shape which is axially downwardly tapered. By pushing the retention ring 42 axially downwardly, a lower section of the retention ring 42 assumes a radially constricted position/shape in the locking state (not shown in Fig. 4C). The lower section of the retention ring 42 engages a lower section of the head 61 such that the head 61 is non-removable in the locking state. In addition, the engagement surface 411 of the fixation cap 41 is clamped between the head 61 and the retention ring 42 which circumferentially encloses the fixation cap 41.

An upper section of the retention ring 42 may be pressed radially outwardly by the engagement surface 411 when the fixation cap 41 is advanced axially downwardly. The fixation cap 41 may engage an upper section of the head 61 in this manner frictionally.

Engaging the upper section and the lower section of the head 61 of the bone anchoring element 6 may fix a spatial axis of the bone anchoring element 6, i.e. the bone anchoring element 6 can no longer be pivoted polyaxially in the receiving chamber 416.

## Claims

1. A coupling unit (101) for coupling a longitudinal element (5) to a bone anchoring element (6), in particular for a screw system (201), preferably a screw system (201) for an intra-vertebral implant, comprising:
- A tulip element (2) which has an axially extending through hole (3) including
o a receiving area (33) for receiving a head (61) of the bone anchoring element (6), in particular a bone screw,
o an upper section (34), preferably having an inner thread (341), and
o at least one aperture (31, 32) in the upper section (34) for receiving the longitudinal element (5),
- A locking mechanism (4) comprising a radially expandable retention ring (42) and a fixation cap (41) which is placeable in the receiving area (33), the retention ring (42) having a retention surface for axially retaining the head (61) of the bone anchoring element (6) and a passage (422) for passing through the head (61) of the bone anchoring element (6), wherein
the locking mechanism (4) is convertible between a pass-through state (43) and a locking state (44) by axially moving the fixation cap (41) along the through hole (3), wherein
- in the pass-through state (43), the retention ring (42) assumes a radially expanded or expandable configuration in an at least partially circumferential space (8) of the receiving area (33) and is configured for passing the head (61) of the bone anchoring element (6) through the passage (422) of the retention ring (42), and
- in the locking state (44), the retention ring (42) of the locking mechanism (4) assumes a radially restricted position such that the retention surface engages at least a lower part of the head (61) of the bone anchoring element (6) such that passing of the head (61) of the bone anchoring element (6) through the passage (422) is prevented, **characterized in that** the fixation cap (41) has a first engagement surface (411) and the retention ring (42) has a second engagement surface (421) configured such that upon axial movement of the fixation cap (41) towards the retention ring (42), the first and second engagement surfaces (411, 421) engage with each other thereby converting the locking mechanism (4) from the pass-through state to the locking state.

2. The coupling unit (101) according to claim 1, wherein the retention ring (42) is arranged in the at least partially circumferential space (8) in an axially immovable position.

3. The coupling unit (101) according to one of the preceding claims, wherein the first and second engagement surfaces (411, 421) include at least one bevel surface (414, 424) inclined with respect to the axis of the coupling unit (101), wherein the first and second engagement surface (411, 421) in particular each include a first and second bevel surface (414, 424), respectively.

4. The coupling unit (101) according to one of the preceding claims, wherein the tulip element (2) comprises an abutment surface (415) engageable with the fixation cap (41) in the receiving area (33) to prevent movement of the fixation cap (41) in an upward axial direction (A) beyond a predetermined axial position.

5. The coupling unit (101) according to one of the preceding claims, wherein the fixation cap (41) is shaped and sized to enclose the retention ring (42) at least partially along a circumferential direction of the retention ring (42) to retain the retention ring (42) in the restricted position in the locking state (44).

6. The coupling unit (101) according to one of the preceding claims, wherein the retention ring (42) is elastically deformable and is radially compressed in the locking state.

7. The coupling unit according to claim 6, wherein the retention ring (42) has an upwardly protruding ridge (423), in particular a cylindrical upwardly protruding ridge (423), which is at least partially enclosed or enclosable along the circumferential direction by the fixation cap (41) in the locking state (44), and wherein the protruding ridge (423) preferably comprises the bevel surface of the second engagement surface (421) which is shaped and sized to engage and move the fixation cap (41) in an upward axial direction when the head (61) is passed through the passage of the retention ring (42) when removing and/or receiving the head (62).

8. The coupling unit (101) according to one of claims 1 to 7, wherein the at least partially circumferential space (8) has an unoccupied section (81) in the pass-through state (43) and the retention ring (42) is radially expandable by advancing and/or retracting the head (61) of the bone anchoring element through the passage (422) such that the retention ring (42) is radially expanded into the unoccupied section (81).

9. The coupling unit (101) according to one of the preceding claims, wherein the retention ring (42) is a circlip or a collet, and in particular comprises at least one gap or split.

10. The coupling unit (101) according to one of the preceding claims, wherein the fixation cap (41) and the at least one aperture (31, 32) overlap at least partially in the axial direction (A) in the pass-through state (43) of the locking mechanism (4).

11. The coupling unit (101) according to one of the preceding claims, wherein the locking mechanism (4) is shaped and sized such that if an axially downwardly directed force is exerted on the fixation cap (41) towards the retention ring (42), the locking mechanism (4) is converted to the locking state (44) and the head (61) cannot be passed through the passage (422), and if no axially downwardly directed force is exerted on the fixation cap (41), the locking mechanism (4) is converted to the pass-through state (43) and the head (61) is configured to be advanced and/or retracted through the passage (422).

12. The coupling unit (101) according to one of the preceding claims, wherein the fixation cap (41) has an axial through hole (412) such that a driver for the head (61) of the bone anchoring element (6) is insertable and/or retractable through the axial through hole (412).

13. The coupling unit (101) according to one of the preceding claims, wherein the fixation cap (41) has a receiving chamber (416) which includes an at least partially spherical inner surface for at least partly frictionally engaging at least an upper part of the head (61) of the bone anchoring element (6).

14. The coupling unit (101) according to one of the preceding claims, wherein the upper section has at least two apertures (31, 32) for receiving the longitudinal element (5) which are arranged on opposing sides of the tulip element (2) and preferably axially extend to an end of the upper section (34).

15. The coupling unit (101) according to one of the preceding claims, wherein in the pass-through state (43), the locking mechanism (4) is configured for engaging the head (61) of the bone anchoring element (6) such that the head (61) of the bone anchoring element (6) is tiltable polyaxially, and wherein the locking mechanism (4), in particular the fixation cap (41) and/or the retention ring (42), frictionally engages the head (61) of the bone anchoring element (6) in the locking state (44) such that of bone anchoring element (6) is non-tiltable.

16. A screw system (201), in particular for an intra-vertebral implant, comprising at least one coupling unit (101) according to one of the preceding claims and at least one bone anchoring element (6), in particular at least one bone screw.

17. The screw system (201) according to claim 15, further comprising at least one longitudinal element (5) which is shaped and sized to be received laterally in the through hole (3), in particular through the at least one aperture (31, 32) .

18. The screw system according to one of the claims 15 or 16, further comprising a set screw (7) which comprises an outer thread (71) which is insertable into the inner thread (341) of the tulip element (2) for exerting a downward axial force on the longitudinal element (5) towards the fixation cap (41) such that the locking mechanism (4) is converted from the pass-through state (43) to the locking state (44).
